# EUROPEAN PATENT APPLICATION

(11) **EP 2 009 095 A1**
(43) Date of publication of application: **31.12.2008**
(21) Application number: 07012698.2
(22) Date of filing: 28.06.2007
(51) Int. Cl.: C12N 5/06, A61K 48/00

(54) **Method of generating glucose-responsive cells**

(71) Applicant: Innovalor AG, 6442 Gersau (CH)
(72) Inventor: Poppe, Monika, 8106 Adilkon (CH); Dieterlen, Maja-Theresa, 04277 Leipzig (DE)
(74) Representative: Schalch, Rainer

(57) **Abstract**

The present invention provides an improved method for generating cells. The method comprises differentiation of neuronal progenitor cells and transfecting of either already differentiated or progenitor cells to generate certain cells useful for the treatment of an illness such as diabetes.

## Description

The present invention provides an improved method for generating cells. The method comprises differentiation of neuronal progenitor cells and transfecting of either already differentiated or progenitor cells to generate certain cells useful for the treatment of an illness such as diabetes.

Diabetes mellitus is one of the most frequent diseases affecting approximately 180 Million patients worldwide. There is a rapid increase in prevalence with an expected rise to 366 Million patients in 2030 (Wild S, et al. Diabetes Care 2004; 27: 1047-1053).

The islets of Langerhans in the pancreas contain endocrine cells that produce and secrete insulin, amylin and glucagon. These hormones help maintain normal blood glucose levels within a remarkably narrow range. Among the islet cells are beta-cells which produce and secrete insulin. Insulin production and secretion by the beta-cells is controlled by blood glucose levels. Insulin release increases as blood glucose levels increase. Insulin promotes the uptake of glucose by target tissues and this prevents hyperglycemia by shuttling glucose into tissues for storage.

Beta-cell dysfunction and the concomitant decrease in insulin production can result in diabetes mellitus. In type I diabetes the beta-cells are completely destroyed by the immune system resulting in an absence of insulin producing cells (Physician's Guide to Insulin Dependent [Type I] Diabetes Mellitus: Diagnosis and Treatment, American Diabetes Association, 1988).

Type I diabetes mellitus is an autoimmune disorder with degeneration of insulin-producing pancreatic islet cells resulting in an absolute insulin deficiency at the onset of clinical symptoms. Type I diabetes is also known as insulin-dependent diabetes, childhood diabetes or juvenile-onset diabetes - characterized by a loss of the insulin-producing beta-cells of the islets of Langerhans of the pancreas leading to a deficiency of insulin. Sensitivity and responsiveness to insulin are usually normal especially in the early stages. This type comprises up to 10 % of total cases in North America and Europe though this varies by geographical location. This type of diabetes can affect children or adults but has traditionally been termed "juvenile diabetes" because it represents a majority of cases of diabetes affecting children. The most common cause of beta-cell loss leading to type I diabetes is autoimmune destruction accompanied by antibodies directed against insulin and islet cell proteins. The principal treatment of type I diabetes even from the earliest stages is replacement of insulin. Without insulin ketosis and diabetic ketoacidosis can develop and coma or death will result.

Currently type I diabetes can be treated only with insulin with careful monitoring of blood glucose levels using blood testing monitors. Emphasis is also placed on lifestyle adjustments (diet and exercise). Apart from the common subcutaneous injections it is also possible to deliver insulin via a pump which allows infusion of insulin 24 hours a day at preset levels and the ability to program a push dose (a bolus) of insulin as needed at meal times. This is at the expense of an indwelling subcutaneous catheter. It is also possible to deliver insulin via an inhaled powder.

Type II diabetes mellitus - also known as adult - onset diabetes, maturity - onset diabetes or non-insulin dependent diabetes mellitus - is due to a combination of the defective insulin secretion and defective responsiveness to insulin (often termed insulin resistance or reduced insulin sensitivity), almost certainly involving the insulin receptor in cell membranes. In early stages the predominant abnormality is reduced insulin sensitivity characterized by elevated levels of insulin in the blood. In the early stages hyperglycemia can be reversed by a variety of measures and medications that improve insulin sensitivity or reduce glucose production by the liver, but as the disease progresses the impairment of insulin secretion worsens, and therapeutic replacement of insulin often becomes necessary. As Type II diabetes is a progressive disease, beta-cell function will deteriorate despite ongoing treatment with any presently available agent.

Thus beta-cells are absent in people with type I diabetes and are functionally impaired in people with type II diabetes.

Beta-cell dysfunction is currently treated in several different ways. In the treatment of type I diabetes (or the late stages of type II diabetes) insulin replacement therapy is used. Insulin therapy - although life saving - does not restore normoglycemia even when continuous infusions or multiple injections are used in complex regimes. For example postprandial levels of glucose continue to be excessively high in individuals on insulin replacement therapy. Thus insulin therapy must be delivered by multiple daily injections or continuous infusion and the effects must be carefully monitored to avoid hyperglycemia, hypoglycemia metabolic acidosis and ketosis. Despite all efforts in improving the insulin replacement strategies, there is a huge risk for all patients not only to sustain high or low glucose related episodes with loss of consciousness or seizures but also to develop long term complications resulting in heart attacks, stroke, blindness, kidney disease, chronic wounds, etc.

Clinical trials demonstrate that tight glucose regulation can prevent the development of diabetic complications but attempts to achieve this regulation be exogenous insulin administration are only partially successful.

Recent evidence suggests that islet cell transplantation with improved systemic immunosuppression may provide a short term durable remission in insulin requirements in type I diabetics (Shapiro et al., NEJM 2000; 343: 230-238; Ryan et al., Diabetes 2001; 50:710-719). Replacements of beta-cells with pancreatic transplants are also reported by Scharp et al. (Transplant 1991, 51:76) and Warnock et al. (Diabetologia 1991, 34:55). Such transplants however, - as the vast majority of other human diseases amenable to treatment by tissue replacement - require finding a matching donor, surgical procedures for implanting the harvested tissue and graft acceptance. Although there have been improvements in islet cell transplantation (s. Shapiro et al, 2000; Ryan et al., 2001) host versus graft reactions limit the function of such transplanted cells and require - after transplantation in a person with type I diabetes - ongoing immunosuppression therapy because cell surface antigens on the beta-cells are recognized and attacked by the same processes that destroyed the beta cells originally. Immunosuppressive drugs such as Cyclosporin A, involve major side effects including the increase in potential for infection. Transplantation therefore can result in numerous complications.

Therefore, efforts have been made to explore alternative tissue sources. Most efforts focus on generating islet or other insulin-producing cells from related tissues or stem cells. Although some of these avenues appear promising (e. g. the generation of islet cells from embryonic stem cells; Blyszczuk and Wobus, Methods Mol Biol 2006, 330: 373-385), immunosuppression would still remain an issue. Such approaches would mostly require allotransplantation. Islet cells present all major immunogenic antigens, inducing major immune responses. Even if the patients own cells were available (e. g. after conversion of bone marrow stem cells) in respect to treat type I diabetes, immunosuppression would still remain necessary since all precursors of insulin would be produced and then function as antigens for autoimmune antibodies.

It is therefore an object of the present invention to overcome at least some of the difficulties or deficiencies associated with the prior art.

The inventors of the present application surprisingly found that neural precursor cells (NPCs) converted into glucose sensitive neurons release heterologously expressed proteins in a glucose-dependent manner with low release of such proteins at low glucose concentrations and a marked increase at concentrations above normal serum glucose concentrations (~ 7 mM). In addition, these cells express neuronal markers such as β-tubulin or MAP-2 as well as a variety of "glucose sensing" proteins such as glucokinase or glucose transporters.

The present invention therefore relates to a neuron comprising a cDNA encoding a protein, wherein the neuron is capable of secreting said protein in a glucose-dependent manner.

A "Neural precursor cell", "neuronal progenitor cell" or "NPC", as used herein, is a cell that is capable of self-renewing and of differentiating into glial cells and neurons. Usually, non-differentiated NPCs express the marker molecules nestin, CD15, CD56, CD90, CD164, and NGFR, whereas they do not express CD45, CD105 (endoglin), CD109, and CD140b (PDGF-RB) [Vogel, W. et al., Heterogeneity among human bone marrow-derived mesenchymal stem cells and neural progenitor cells, J. Haematol., 2003, 88, 126-133; the disclosure of which is incorporated herein by reference]. The term "self-renewing" refers to the capability of a cell to undergo mitosis and to divide to produce two daughter cells.

"Neuronal cells" are cells that express the marker protein(s) neurofilament, microtubule-associated protein-2 (MAP-2) and/or β-tubulin III (Tuj1). They may further express the marker proteins neurofilament, calbindin and tau.

The terms "neurons" and "mature neurons" as used herein denote post-mitotic neuronal cells. It is generally accepted that mature neurons are postmitotic. To confirm maturity and postmitotic status usually the expression of calbindin is considered a specific marker, while doublecortin serves as marker for developing neuronal cells that may still undergo cell division. Neurons do not express the markers nestin and doublecortin.

Neurons offer huge advantages in respect to transplantation:
1. Neurons are not immunogenic. Neurons only express minor densities of major histocompatibility proteins (no MHC-II, little MHC-I). Thus, these cells are unlikely to be subject to major reactions of the host immune system.
2. Neurons exhibit profound longevity. Therefore, neurons are likely to survive for many years following transplantation. This will ensure long-lasting effects of transplanted cells.
3. Neurons are post-mitotic. The lack of cell divisions will ensure that heterologously expressed genes are unlikely to be eliminated from the transfected neurons. In addition, tumor formation is unlikely to occur in post-mitotic cells.
4. Neurons express an intact protein secretion machinery which includes the packaging of peptides and proteins into synaptic vesicles and its strongly regulated exocytosis.

The neuron of this invention expresses at least one neuronal marker. For example, the neuron expresses one or more of the neuronal markers Tuj1, MAP-2 and neurofilament. Preferably, the neuron of the invention expresses Tuj1 and/or MAP-2. Most preferably, the neuron of the invention expresses MAP-2. The expression of markers can be determined by immunocytochemical or by nucleic acid-based techniques (e.g. RT PCR), as described in the examples herein.

The neuron of the present invention is capable of expressing and secreting the protein encoded by the cDNA in a glucose-dependent manner. This means in the sense of this application that the amount of said protein secreted by the neuron in the presence of 7.5 mM glucose is greater than the amount of said protein secreted by the cell in the presence of 2.5 mM glucose. The ratio between the amount of protein secreted by the neuron in the presence of 7.5 mM glucose and the amount of protein secreted by the neuron in the presence of 2.5 mM glucose is preferably at least 1.5, more preferably at least 5, even more preferably at least 10, most preferably at least 20.

In one embodiment, the neuron essentially does not secrete the above-mentioned protein in the presence of a glucose concentration of 3 mM or less, e.g. in the absence of glucose.

The amount of a given protein expressed or secreted by a population of neurons can be determined by methods that are known to those of ordinary skill. For example, the concentration of proteins secreted into the culture medium by neurons that are cultured *in vitro* can be determined by immunological methods using antibodies directed against the protein to be detected, Such methods may be direct or indirect immunoassays. These assays include but are not limited to competitive binding assays, non-competitive binding assays, radioimmunoassays, enzyme-linked immunosorbent assays (ELISA), sandwich assays, precipitin reactions, gel diffusion immunodiffusion assays, agglutination assays, fluorescence immunoassays, chemoluminescence immunoassays, immunoPCR immunoassays, protein A or protein G immunoassays and immunoelectrophoresis assays.

The immunoassay comprises the use of a polyclonal or monoclonal antibody capable of binding to the protein to be detected. As used herein, the term "antibody" designates an immunoglobulin or a derivative thereof having the same binding specificity. The antibody according to the invention may be a monoclonal antibody or an antibody derived from or comprised in a polyclonal antiserum, monoclonal antibodies are preferred. The term "antibody", as used herein, further comprises derivatives such as Fab, F(ab')₂, Fv or scFv fragments: see, for example Harlow and Lane, "Antibodies, A Laboratory Manual" CSH Press 1988, Cold Spring Harbor N.Y. Fab, F(ab')₂ and Fv fragments fragments can be obtained by digesting a complete antibody with papain, pepsin, etc. in the conventional manner. The antibody or the derivative thereof may be of natural origin or may be (semi)synthetically produced. Such synthetic products also comprises non-proteinaceous or semi-proteinaceous material that has the same or essentially the same binding specificity as the antibody of the invention. Such products may, for example be obtained by peptidomimetics.

In the ELISA method, an antibody, preferably a monoclonal antibody, or a fragment thereof against the protein is first immobilized on a carrier as a primary antibody. Preferred carrier is a solid carrier, such as an ELISA plate container molded from a carrier polymer such as styrene or polystyrene. The monoclonal antibody or its fragment can be immobilized by, for example, dissolving the monoclonal antibody or its fragment in a buffer such as a carbonate buffer or a borate buffer followed by the adsorption onto the carrier.

Separately, an antibody (a monoclonal antibody or a polyclonal antibody, or a fragment thereof) used as the secondary antibody is labeled preferably with a non-radioactive label. Enzyme labels, fluorescent labels, light emission labels, etc. can be used as the non-radioactive label. It is preferred that an enzyme label such as alkaline phosphatase, β-galactosidase or horse radish peroxidase be used.

The neuron of the invention preferably expresses at least one glucose-sensing marker selected from the group consisting of GLUT2, glucokinase and GLP-1 receptor. The neuron may express one, two or three of these markers.

In another preferred embodiment, the protein expressed and secreted by the cell is insulin, preferably human insulin. Insulin consists of two polypeptide chains, commonly termed the A chain and the B chain, with three disulfide bridges formed between half-cystine residues; two of these bridges are inter-chain, and the third is intra-chain within the A chain. Insulin ist biosynthesized by post-translational modification of its single-chain precursor preproinsulin. Human preproinsulin consists of 110 amino acids. In preproinsulin, the N-terminal residues 1-24 are the signal sequence. When this is removed, proinsulin is formed. In the Golgi apparatus, prohormone convertases PC2 and PC3 cleave proinsulin on the carboxyl side of a pair of basic amino acid residues (Arg55-Arg56 and Lys88-Arg89). This excises the C peptide (residues 57-87), leaving the A and B chains (Arg55 and Arg56 must first be cleaved from the B chain to for the mature B chain to be formed). Residues 55-87 are known as the connecting peptide. In human insulin, the A chain corresponds to amino acids 90-110, and the B chain corresponds to amino acids 25-54 of the preproinsulin sequence. The term "insulin" as used herein includes mature insulin, proinsulin, preproinsulin and variants and derivatives thereof.

In the case of insulin, the neurons of the invention may secrete about 0.1 to about 10 international units of insulin/10⁶ cells/day. Preferably, they secrete about 0.5 to about 8, more preferably about 1 to about 5 international units of insulin/10⁶ cells/day.

The cDNA comprised in the neuron of the invention preferably encodes insulin. The cDNA may lack a DNA sequence encoding the C-peptide of insulin. This offers the advantage that no immunogenic C-peptide can be expressed and secreted by the neurons of the invention. Preferred are cDNAs encoding single-chain insulin analogues as described in EP1193272A1 or W09634882A1. The insulin analogues, the nucleic acids encoding them and the vectors disclosed in EP1193272A1 or WO9634882A1 are incorporated herein by reference. An example for a nucleic acid encoding a sinle-chain analogue can also be found under GenBank accession number BD181089 (GI:30792007), see SEQ ID NOs: 17 and 18.

In one embodiment, the cDNA is a recombinant DNA. The term "recombinant" means, for example, that a nucleic acid sequence is made by an artificial combination of two otherwise separated segments of sequence, e.g., by chemical synthesis or by the manipulation of isolated nucleic acids by genetic engineering techniques. In another embodiment, the cDNA is fused to a heterologous sequence. The cDNA is usually operably linked to a suitable promoter sequence.

Another aspect of this invention is a population of cells, in which at least 10% of the cells are neurons as described hereinabove. Preferably, at least 20%, more preferably at least 30%, most preferably at least 40% of the cells in the population are neurons as described hereinabove.

In the population of cells of this invention, at least 10%, preferably at least 20%, more preferably at least 30%, and most preferred more than 40% of the cells may be positive for expression of the marker protein MAP-2.

In another embodiment, at least 15%, preferably at least 30%, more preferably at least 45% and most preferred more than 60% of the cells in the population of cells of this invention are positive for expression of β-tubulin III (TUJ1).

In yet another embodiment, at least 20%, preferably at least 30%, more preferably at least 50%, and most preferred more than 60% of the cells in the population of cells of this invention are positive for expression of insulin.

In a particular embodiment, the neurons of this invention do not express MHC-I molecules on their surface. According to that embodiment, at least 20%, preferably at least 40%, more preferably at least 60%, and most preferred more than 90% of the cells in the population of cells of this invention are negative for expression of MHC-I molecules.

The present invention further relates to a method for generating neurons that are capable of expressing a protein in a glucose-dependent manner, comprising the steps of
(a) culturing neural precursor cells in the presence of at least one factor capable of promoting islet cell development; and
(b) transfecting the cells with cDNA encoding the mentioned protein.

The method may further comprise the step of providing neural precursor cells (NPCs).

Isolation and culturing of NPCs from rodent brain has been reported in the state of the art (Daadi und Weiss, Generation of tyrosine hydroxylase producing neurons from precurors of the embryonic and adult forebrain, J Neurosci 1999, 19: 4484-4497; Liepelt et al., Differentiation potential of a monoclonal antibody-defined neural progenitor cell population isolated from prenatal rat brain by fluorescence-activated cell sorting, Brain Res Dev Brain Res 1999, 51: 267-278).

NPCs can be isolated from fetal (embryonic) or adult brain or spinal cord preparations. If an adult donor is used NPCs are preferably isolated from subventricular or hippocampal brain regions. In case of humans, the term "adult" preferably refers to an individual at an age of at least about 16 years, e.g., about 16 years to about 45 years, more preferably at least about 18 years, e.g., about 18 years to about 30 years. However, also individuals at an age of less than 16 years or greater than 45 years may be suitable donors.

NPCs were successfully isolated from various parts of brain. NPCs are abundant in fetal (embryonic) brain tissue. Preferably, NPCs are isolated from fetal frontal cortex or midbrain tissue. Most efficiently, NPCs are prepared from human fetal brain tissue, 3 - 25 weeks of gestation, preferably 5 - 14 weeks of gestation, most preferably 10-11 weeks of gestation.

Isolation of NPCs from human fetal brain tissue has been described e.g. in Buc-Caron, (Neuroepithelial progenitor cells explanted from human fetal brain proliferate and differentiate in vitro, Neurobiol Dis 1995, 2: 37-47), Svendsen CN et al. (Long-Term Survival of Human Central Nervous System Progenitor Cells Transplanted into a Rat Model of Parkinson's Disease, Exp Neurol 1997, 148: 135-146), Sah et al. (Biopotent progenitor cell lines from the human CNS , Nat Biotechnol 1997, 15: 574-580), Chalmers-Redman et al. (In vitro propagation and inducible differentiation of multipotential progenitor cells from human fetal brain, Neuroscience 1997, 76: 1121-1128) and Schwarz SC et al. (Parkinsonism Relat Disord. 2006, 12(5): 302-308). The disclosure of these documents is incorporated herein by reference. The technique of preparation of brain tissue and isolation of NPCs can be adapted from these protocols.

It is also possible that the NPCs are generated from other stem cells, e.g. mesenchymal stem cells or embryonic stem cells. The mesenchymal cells can be isolated from bone marrow, preferably adult bone marrow, more preferably human adult bone marrow (e.g. bone marrow stromal cells). Alternatively, the mesenchymal stem cells can be isolated from umbilical cord blood, preferably human umbilical cord blood, or from adipose tissue. Methods of isolating mesenchymal stem cells from various sources are known to those skilled in the art. They can be converted to NPCs by methods known in the art (see e.g. WO 2005/017132 A1 filed by NeuroProgen GmbH Leipzig, and references cited therein; Hermann et al. J Cell Sci., 2004, 117(Pt19):4411-4422). Using these sources of stem cells, it is possible to use autologous cells in transplantation by isolating stem cells from the body of an individual (e.g. a person suffering from Parkinson's Disease), converting the stem cells into NPCs, culturing the NPCs as described in this application, and transplanting the autologous cells into the same individual. This embodiment has the great advantage that immunological complications such as rejection of the grafted cells can be avoided.

In a particular embodiment of the invention, the NPCs are cultured, expanded and/or converted under an atmosphere having an oxygen concentration of less than 20% (v/v). Preferably, the oxygen concentration is less than 15% (v/v), more preferably less than 10% (v/v), still more preferably less than 5% (v/v), most preferably about 3% (v/v). The minimum oxygen concentration may be 0.1 % (v/v) or 1% (v/v). The cells may be cultured under an atmosphere of 5% (v/v) CO₂, 92% (v/v) N₂ and 3±2 % (v/v) O₂. The culturing under an reduced oxygen may be performed at any time of the culturing, for example when proliferating and/or when differentiating the NPCs as described herein.

According to a specific embodiment of the method of the invention the culture medium contains an effective Ca²⁺ concentration of less than 1.0 mmol/l, preferably less than 0.5 mmol/l, more preferably less than 0.1 mmol/l. Preferably, the total concentration of Ca²⁺ ions in the culture medium is identical to the effective concentration. If needed, a masking of Ca ions through suitable masking agents which decrease the concentration of free Ca ions may occur. For this purpose, chelating agents like EGTA, EDTA, Kronenether and others may be used. If desired, the culture medium may be free of Ca²⁺ ions apart from unavoidable contaminations; preferably, the medium is not Ca²⁺ free. A minimum amount of Ca²⁺ ions of 0.001 to 0.1 mmol/l, particularly 0.01 or 0.05 to 0.1 mmol/l culture medium, may be employed. Furthermore, the culture medium may comprise Mg ions in only low concentration, or it is free of Mg ions, apart from unavoidable contaminations. The Mg concentration of the culture medium can be less than 2 mmol/I culture medium, preferably less than 0.6 mmol/I or less than 0.1 mmol/I culture medium (See WO03/010304 A2 by NeuroProgen Leipzig GmbH). The indicated calcium ion concentrations may be present at any time of the culturing, for example when proliferating and/or when differentiating the NPCs as described herein.

It is further preferred that the NPCs are cultured at a temperature of from 35 to 39°C, more preferably at a temperature from 36 to 38°C, most preferably at 37°C.

Prior to step a), the NPCs may be expanded for some time, e.g. from 1 min to several months, or from 12 hours to 7 days. Methods of expanding NPCs and suitable culture media are disclosed in e.g. WO 00/78931 A2, filed by NeuroProgen GmbH; or WO03/010304 A2 by NeuroProgen Leipzig GmbH.

The method of the invention comprises the step of culturing neural precursor cells in the presence of at least one factor capable of promoting islet cell development. The factor capable of promoting islet development may be selected from the group consisting of bFGF, Prolactin, Betacellulin, Glucagon, GLP-1 (Glucagon-like protein 1), Exendin-4, Nicotinamid, N2-supplement, retinoic acid, glucose waves, withdrawal of mitogens, arginin and IGF1.

"Glucose waves" as used herein means reducing the glucose concentration in the culture medium, followed by increasing the glucose concentration in the culture medium. In other words, treatment with glucose waves comprises exposing the cells successively to:
(1) a first high glucose concentration,
(2) a first low glucose concentration,
(3) a second low glucose concentration which is higher than the first low glucose concentration [(2)], and
(4) a second high glucose concentration.

The glucose concentration in (1) may range from about 16 mM to about 17,5 mM. The glucose concentration in (2) may range from about 4 mM to about 6 mM. The glucose concentration in (3) may range from about 7 mM to 8 mM. The glucose concentration in (4) may or may not differ from that in (1), and preferably ranges from about 15 mM to about 17,5 mM.

The preferred concentration ranges for the factors recited supra are :

| **Substance** | **range (min-max)** | **preferred range** | **most preferred concentration** |
|---|---|---|---|
| bFGF | 10 ng/ml - 50 ng/ml | 15 ng/ml - 30 ng/ml | 20 ng/ml |
| EGF | 10 ng/ml - 50 ng/ml | 15 ng/ml - 30 ng/ml | 20 ng/ml |
| Prolactin | 10 ng/ml - 5 µg/ml | 50 ng/ml - 500 ng/ml | 250 ng/ml |
| Betacellulin | 1 ng/ml - 1 µg/ml | 10 ng/ml - 100 ng/ml | 50 ng/ml |
| Glucagon | 0,1 µg/ml - 5 µg/ml | 0,5 µg/ml - 2 µg/ml | 1 µg/ml |
| GLP-1 | 50 nM - 250 nM | 75 nM - 200 nM | 100 nM |
| Exendin-4 | 1 nM - 100 nM | 5 nM - 20 nM | 10 nM |
| Nicotinamid | 1 mM - 50 mM | 5 mM - 20 mM | 10 mM |
| Retinoic acid | 1 µM - 10 µM | 1 µM - 5 µM | 2 µM |
| Arginin | 1 mM - 100 mM | 5 mM - 20 mM | 10 mM |
| IGF-1 | 1 nM-50nM | 5 nM - 20 nM | 10 nM |
| N2-Supplement | 0,5 % - 3 % | 0,5 % - 2 % | 1 % |
| B27-Supplement | 1% - 6% | 1% - 4% | 2% |

In a first embodiment, step a) comprises culturing the NPCs in the presence of bFGF, N2-Supplement, B27-Supplement, Nicotinamid und retinoic acid. Preferably, the culture medium additionally contains one, two or three factors selected from prolactin, betacellulin, and GLP-1. Optionally, one or more factors selected from glucagon, exendin-4, IGF-1 und arginine may be added to the culture medium.

The converted glucose sensitive neurons can be transduced to express one or multiple foreign genes. Preferably, the cells are transduced with a DNA encoding insulin. More preferably, the DNA encodes an insulin analogue described hereinabove. Preferred cDNAs, vectors and constructs are described in e.g. EP1193272A1 or WO9634882A1.

Gene transfer may be achieved via plasmid transfection, nucleofection, electroporation, direct transfer of DNA/RNA or by using a viral vector. All methods of gene transfer have been reported in great detail. The preferred method of gene transfer is nucleofection (Amaxa), providing sufficient efficiency and limited toxicity. A suitable protocol for nucleofection may be as follows:
- one nucleofection > use about 4-5x10⁶ cells (minimal cell number: 1x10⁶ cells; maximum cell number: 6x10⁶)
- sample contains 2-20 µg plasmid DNA (in 1-5 µl H₂O or TE buffer)
- select program A-33 nr O-05 according tn the manufacturer s product information

control 1: Recommended amount of cells in Nucleofector Solution with DNA but without application of the program (alternatively: untreated cells) (Cells + Solution + DNA - program)
control 2: Recommended amount of cells in Nucleofector Solution without DNA with application of the program (Cells + Solution - DNA + program)
   Further details of the protocol can be found are given in the manufacturer's product information or instruction manual.

The vector to be used may include the following elements.:
Promoter, e.g. chicken-beta-actin-promoter: (See for example EP0351586A1, the disclosure of which is incorporated herein by reference; or the DNA sequence encoding chicken beta actin gene promoter having GenBank accession number E02199 (GI:2170437), incorporated herein by reference.
IRES-Site: e.g. the internal ribosome entry site of the encephalomyocarditis virus (ECMV) between the multiple cloning site (MCS) and the enhanced green fluorescent protein (EGFP) coding region; this permits both the gene of interest (cloned into the MCS) and the EGFP gene to be translated from a single bicistronic mRNA.
EGFP: enhanced green fluorescent protein = a red-shifted variant of wild-type GFP which has been optimized for brighter fluorescence and higher expression in mammalian cells; preferably, however, the vector contains a DNA sequence encoding insulin as described hereinabove.
Antibiotics resistance for selection, e.g. Kan^{r}/Neo^{r}: neomycin-resistance cassette (Neo^{r}), allows stably transfected eukaryotic cells to be selected using G418
SV40 enhancer: to elevate the basal level of SIA expression
Albumin leader sequence at the SIA: to facilitate the secretion of SIA

The method of the present invention can be carried out in vitro or in vivo, wherein it is also possible that some parts/steps of the method are carried out in vitro and some parts/steps are carried out in vivo. It is preferred that the method is carried out in vitro.

It is also possible to add one or more additional steps to the method of the present invention.

The steps of the method of the present invention can be carried out in any suitable order wherein it is preferred to carry out step a) before carrying out step b).

The present invention involves the conversion of NPCs or other cells to differentiate into glucose sensitive neurons. NPCs may be converted by withdrawal of mitogens (e. g. EGF and bFGF) and the addition of factors that promote islet cell development not only induce neurogenesis compared to gliogenesis but also render the resulting neurons sensitive to glucose. Insulin-producing neurons disclosed herein are positive for insulin and for one or more markers for glucose sensing (Glut2, glucokinase and GLP-1-receptor). The glucose sensing mechanism is intact and insulin secretion can be induced by physiologically high glucose levels (more than 7 mM). At low glucose concentrations (less than 2,5 mM) insulin secretion is suppressed. Additionally, insulin producing neurons can induce vesicular packaging and controlled exocytosis of foreign genes in a comparable glucose dependent manner like it is described for insulin.

The invention further relates to a population of cells obtainable or obtained by a method desribed herein.

Another aspect of the present invention is the use of the neurons of the invention for the manufacture of a pharmaceutical composition. The pharmaceutical composition is preferably for treating diabetes which is - most preferred - diabetes type I. A pharmaceutical composition in the sense of the present invention is any composition which is suitable for pharmaceutical purposes and includes compositions for transplantation purposes. Compositions for transplantation purposes are particularly preferred.

This invention mainly focuses on the generation of glucose sensitive neurons derived from fetal human neural stem cells. It has proven difficult to culture NPCs over longer periods of time and at the same time to retain the differentiation potential of the NPCs. The rate of proliferation of human NPCs is limited (Ostenfeld et al., Exp Neurol. 2000, 164(1):215-226). Thus, extended expansion and modification of such cells is limited. Using low oxygen conditions, the applicants have for the first time developed culture systems that allow for expansion of > 50 passages without affecting the potential of such precursor cells to differentiate into neurons or glia. Such long-term culture systems also allow for partial differentiation and modulation of such cells via long-term application of novel small molecules (see WO 00/78931 A2, filed by NeuroProgen GmbH). Therefore, the percentage of specific cells (e. g. glucose-sensitive neurons) can be markedly increased.

Taken together, the herein disclosed method of generating neurons that secrete heterologously expressed proteins (e. g. insulin) in a glucose-dependent manner represent a novel and promising approach to replace endogenous insulin-producing pancreatic islet cells. Therefore, these cells are likely to offer a novel strategy to provide efficient and safe treatment to patients suffering from diabetes mellitus.

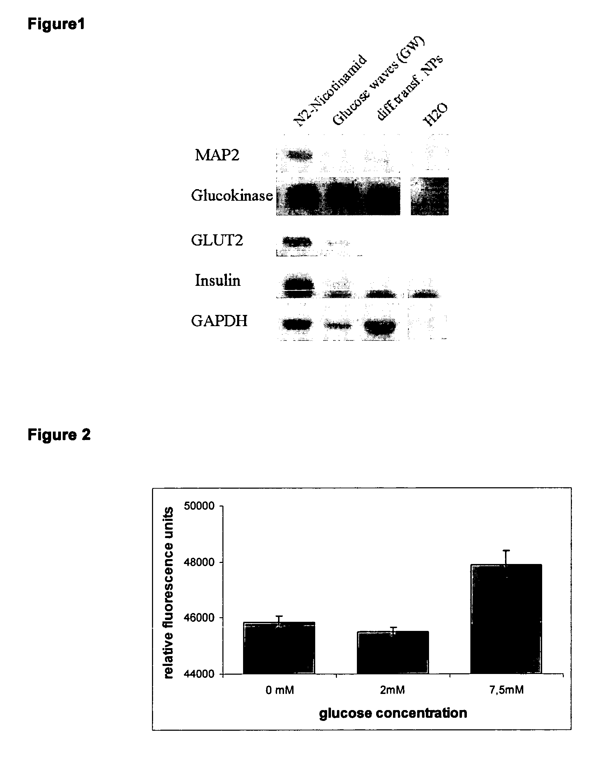

**Figure 1**: Characterization of glucose sensitive neurons by RT-PCR. The neuronal marker MAP2, the glucose-sensing-markers Glucokinase and GLUT2, insulin and the housekeeping gene GAPDH have been detected. H₂O acts as a negative control. See that glucose waves (alone) are not efficient to induce expression of MAP2, GLUT2 and insulin. "diff. transf. NPs" = differentiated transfected neuronal precursors / progenitors
**Figure 2**: EGFP-detection after glucose-stimulation of EGFP-transfected insulin-producing neurons. Stimulation of glucose-responsive neurons with 7,5 mM glucose resulted in secretion of the transfected protein (EGFP), whereas stimulation with 2 mM glucose did not induce any protein secretion.

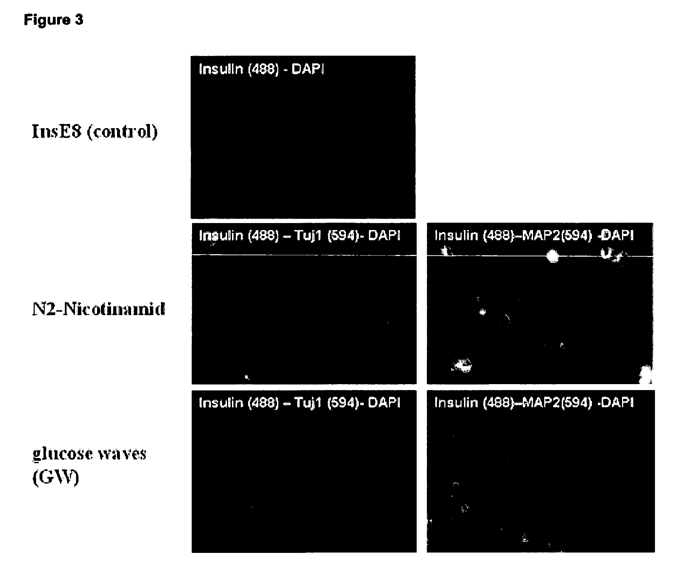

**Figure 3****:** Immunocytochemical staining of differentiated insulin-producing neurons. Pictures show double-stainings of the neuronal markers MAP2 or Tuj1 (red fluorescence, Alexa594) and insulin (green fluorescence, Alexa488). Nuclei were counterstained with DAPI. We showed specific insulin-staining by staining the rat insulin-producing cell line InsE8.

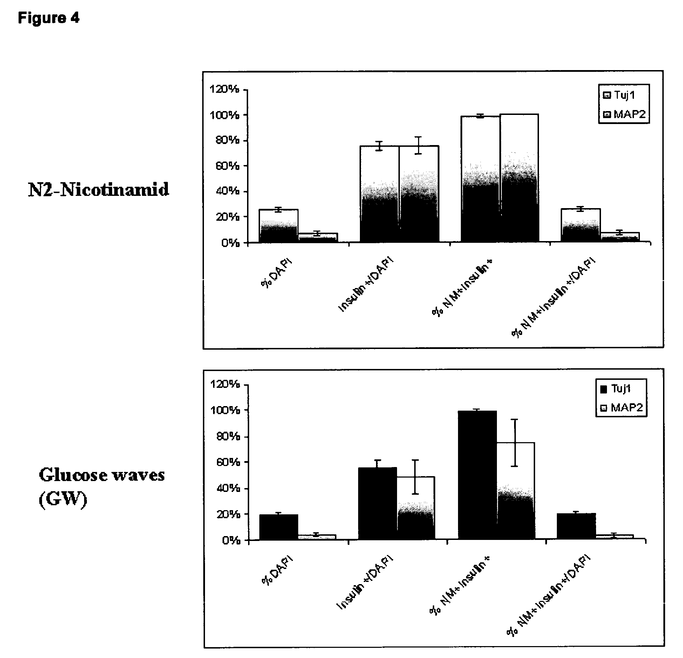

**Figure 4****: I**mmunocytochemical quantification of insulin-producing neurons from different transformation protocols. We showed the percentage of neuronal marker (MAP2 or Tuj1) = NM positive cells to DAPI-positive cells [%DAPI], the percentage of insulin-positive cells to DAPI-positive cells [%Insulin+/DAPI], the percentage of insulin-positive cells at the neuronal population [%NM+Insulin+] and the percentage of insulin-producing neurons to DAPI-positive cells [%NM+Insulin+/DAPI]. Data were shown as mean ± s.e.m.

The invention is illustrated on the basis of the following non-limiting examples:

### Examples

### 1. Material and methods

### Preparation of neuronal progenitor cells

Tissue samples (6-11 weeks post-fertilization) used for the isolation of neuronal progenitor cells are typically harvested from fetal frontal cortex or midbrain by standard micro-dissection procedures. Dissected frontal cortex or midbrain is incubated with Papain-recDNAse for 30 min at 37°C followed by centrifugation and two washing steps with HBSS. Papain is inhibited by incubation with antipain for 30 min at 37°C followed by centrifugation and two washing steps with HBSS. Cells were then homogenized in 1 ml expansion medium and cell counts were designated. According to their cell counts cells were plated onto poly-I-ornithin-fibronectin coated cell culture vessel in serum-free media (1 DMEM : 1 HamsF12, 2% B27-supplement, 20 ng/ml EGF, 20 ng/ml FGF-2, 1% Pen/Strep.

### N2-Nicotinamid differentiation:

Neuronal progenitors were expanded in *expansion medium* I: 1 DMEM High Glucose : 1 Ham'sF12 (both PAA Laboratories, Pasching, Austria), 2% B27 (Invitrogen, Karlsruhe, Germany), 1% Pen/Strep (Invitrogen, Karlsruhe, Germany), 20ng/ml EGF, 20ng/ml bFGF (both Peprotech, Rocky Hill NJ, USA). Cells were expanded for 6-12 weeks (4-12 passages) after preparation before they were used for generation of glucose-responsive cells.

1x10⁷ cells were cultivated for 1 week in *N2-Nic-stage2-medium:* 1 DMEM High Glucose : 1 Ham'sF12, 1% N2-Supplement (Invitrogen, Karlsruhe, Germany), 2% B27, 20ng/ml bFGF, 1 % Pen/Strep.

After 1 week 2-4x10⁵ cells were plated for another 7 days onto poly-L-ornithin-fibronectin-coated 6 well plates in *N2-Nic-stage3-medium:* 1 DMEM High Glucose : 1 DMEM Low Glucose (PAA Laboratories, Pasching, Austria), 1% N2-Supplement, 2% B27, 10mM Nicotinamid (Sigma, Munich, Germany), 2µM retinoic acid (Sigma, Munich, Germany), 1% Pen/Strep. Medium was changed every other day.

The glucose concentrations in the media were as follows:

| | |
|---|---|
| *Expansion medium:* | 17,3 mM (Min: 16 mM; Max: 17,5 mM) |
| *N2-Nic-stage2-medium*: | 17,3 mM (Min: 16 mM; Max: 17,5 mM) |
| *N2-Nic-stage3-medium:* | 15,3 mM (Min: 15 mM; Max: 17,5 mM) |

### Differentiation by glucose waves:

Neuronal progenitors were expanded in expansion medium I: 1 DMEM High Glucose : 1 Ham'sF12 (both PAA Laboratories, Pasching, Austria), 2% B27 (Invitrogen, Karlsruhe, Germany), 1% Pen/Strep (Invitrogen, Karlsruhe, Germany), 20ng/ml EGF, 20ng/ml bFGF (both Peprotech, Rocky Hill NJ, USA).

1x10⁷ cells were cultivated for 2 weeks in gw-stage2-medium: 1 DMEM Glucose-free (PAA Laboratories, Pasching, Austria): 1 Ham's F12, 2% B27, 20ng/ml bFGF, 20ng/ml EGF 1% Pen/Strep. Medium was changed once a week.

After 2 weeks 2-4x10⁵ cells were plated for another 2 weeks onto poly-L-ornithin-fibronectin-coated 6 well plates in gw-stage3-medium: 1 DMEM Low Glucose (PAA Laboratories, Pasching, Austria) : 1 Ham'sF12, 2% B27, 2µM retinoic acid (Sigma, Munich, Germany), 1% Pen/Strep. Medium was changed every other day.

After 2 weeks cells were cultivated for 6 days with gw-stage4-Medium: 1 High Glucose DMEM : 1 Low Glucose DMEM, 2% B27, 1% Pen/Strep). Medium was changed every other day.

The glucose concentrations in the media were as follows:

| | |
|---|---|
| *Expansion medium:* | 17,3 mM (Min: 16 mM; Max: 17,5 mM) |
| *GW-stage2-medium:* | 5 mM (Min: 4 mM; Max: 6 mM) |
| *GWstage3-medium:* | 7,5 mM (Min: 7 mM; Max:8 mM) |
| *GWstage4-medium:* | 15,3 mM (Min: 15 mM; Max: 17,5 mM) |

### N2-Nicotinamid differentiation including glucose waves:

Neuronal progenitors were expanded in *expansion medium* I: 1 DMEM High Glucose : 1 Ham'sF12 (both PAA Laboratories, Pasching, Austria), 2% B27 (Invitrogen, Karlsruhe, Germany), 1% Pen/Strep (Invitrogen, Karlsruhe, Germany), 20ng/ml EGF, 20ng/ml bFGF (both Peprotech, Rocky Hill NJ, USA). Cells were expanded 6-12 weeks (4-12 passages) after preparation before they were used for generation of glucose-responsive cells.

1x10⁷ cells were cultivated for 1 week in *N2Nic+gw-stage2-medium:* 1 DMEM Glucose-free (PAA Laboratories, Pasching, Austria): 1 Ham's F12, 1% N2-Supplement (Invitrogen, Karlsruhe, Germany), 2% B27, 20ng/ml bFGF, 1% Pen/Strep.

After 1 week 2-4x10⁵ cells were plated for another 7 days onto poly-L-ornithin-fibronectin-coated 6 well plates in *N2Nic-gw-stage3-medium:* 1 DMEM High Glucose : 1 DMEM Low Glucose (PAA Laboratories, Pasching, Austria), 1% N2-Supplement, 2% B27, 10mM Nicotinamid (Sigma, Munich, Germany), 2µM retinoic acid (Sigma, Munich, Germany), 1% Pen/Strep. Medium was changed every other day.

| | |
|---|---|
| *Expansion medium:* | 17,3 mM (Min: 16 mM; Max: 17,5 mM) |
| *N2Nic-gw-stage2-medium:* | 5 mM (Min: 4 mM; Max: 6 mM) |
| *N2Nic-gw-stage3-medium:* | 15,3 mM (Min: 15 mM; Max: 17,5 mM) |

### Methods for detection and quantification of insulin-producing neurons:

### RT-PCR:

Total RNA was prepared using the RNeasy Mini Kit (Qiagen, Hilden, Germany). The First Strand cDNA Synthesis Kit (Fermentas, St. Leon-Rot, Germany) with random hexamer primers was used to perform reverse transcription reaction and cDNA synthesis. A standard 25µl PCR reaction was performed using Taq-DNA-Polymerase (Fermentas, St. Leon-Rot, Germany). Primer sequences, annealing temperature, number of cycles and product sizes are listed in Table 1. PCR products were separated on a 1% TAE agarose gel, photographed and analyzed with a UV detection camera system (Multilmage^{™}LightCabinet, Alphalnnotech Corporation).

**Table 1: Primer sequences, PCR conditions and PCR product sizes.**

| **Gene** | **Forward Primer** | **Reverse Primer** | **Annealing-temperature (°C)** | **Numnber of cycles** | **Product Size (bp)** |
|---|---|---|---|---|---|
| Insulin | 5'-TCAGCACCTGTGCGGCTCT CACCT-3' (SEQ ID NO:1) | 5'-CGATGCCACGTTTACCCG GACCC-3' (SEQ ID NO:2) | 55 | 30 | 130 |
| MAP2 | 5'-ATCAAGGCGGAGCAGGGGG AAGGAC-3' (SEQ ID NO:3) | 5'-TGGGGGTGGAGAAGGAGG CAGATT-3' (SEQ ID NO:4) | 60 | 35 | 320 |
| Glut2 | 5'-GGTTTGTAACTTATGCC TAAG-3' (SEQ ID NO:5) | 5'-GCCTAGTTATGCATTGCAG-3' (SEQ ID NO:6) | 60 | 30 | 213 |
| Glucokinase | 5'-GAATACCCCCCAGAGACCT TTTC-3' (SEQ ID NO:7) | 5'-GGTTTCTTCCTGAGCCA GCG-3' (SEQ ID NO:8) | 60 | 30 | 200 |
| GAPDH | 5'-GTCAGTGGTGGACCTGA CCT-3' (SEQ ID NO:9) | 5'-TGCTGTAGCCAAATTCG TTG-3' (SEQ ID NO:10) | 56 | 26 | 250 |
| GLP-1-Rezeptor | 5'-TTCTGCAACCGGACCTT CGA-3' (SEQ ID NO:11) | 5'-ATGAGTGTCAGCGTGGAC TTG- 3' (SEQ ID NO:12) | 55 | 35 | 850 |
| Nestin | 5'-CGTTGGAACAGAGGTTG GAG-3' (SEQ ID NO:13) | 5'-TAAGAAAGGCTGGCAC AGGT-3' (SEQ ID NO:14) | 60 | 35 | 360 |
| Beta-III-Tubulin | 5'-CAACAGCACGGCCATCCAGG- 3' (SEQ ID NO:15) | 5'-CTTGGGGCCCTGGGCCTCCGA- 3' (SEQ ID NO:16) | 55 | 35 | 250 |

### Immunocytochemistry:

Cells were fixed in 4% paraformaldehyde in PBS for 10 minutes at room temperature and washed 3 times with PBS. The cells were incubated in blocking buffer: DMEM; 10% FCS (PAA Laboratories, Pasching, Austria); 0.2% Triton-X-100 (Roth, Karlsruhe, Germany) for 30 minutes at room temperature. After incubation with the primary antibodies for 1 hour at room temperature in blocking buffer, the cells were washed 3 times with blocking buffer followed by incubation with Alexa Fluor-labeled secondary antibodies diluted in blocking buffer. After incubation with secondary antibodies cells were washed 3 times with PBT: 0,1% Tween (Merck, Darmstadt, Germany) in PBS, pH 7,4. The cells were then counterstained with the DNA-binding dye 4'-6'-diamidino-2-phenylindole (DAPI) (2 µg/ml in PBT) for 15 minutes at room temperature and washed twice with PBT. Coverslips were mounted onto glass slides and examined under a fluorescence microscope (Zeiss Axiovert 200). Acquisition of the stained cells was performed using the image-analysis software AxioVision 4 (Zeiss, Jena, Germany).

The following antibodies were used for immunocytochemistry: mouse anti-β-tubulin III antibody (Covance, Princeton, NJ); guinea pig anti-human insulin (Linco Research, St. Charles, Missouri, USA); goat anti guinea-pig Alexa Fluor 488 conjugate (Molecular Probes, Eugene, OR, USA); donkey anti-mouse IgG Alexa Fluor 594 conjugate (Molecular Probes).

### In vitro insulin secretion assay and insulin quantification:

4x 10⁵ cells were incubated with 1 ml of glucosefree DMEM (PAA Laboratories, Pasching, Austria) containing 25 mM glucose for 2 h at 37 °C, 3 % O₂ and 5 % CO₂. Supernatants were then harvested for enzyme-linked immunosorbent assay (ELISA)-based quantification of released insulin using AutoDELFIA^{®}-Kit (PerkinElmer, Wellesley, MA, USA).

These cell culture conditions may be used to determine whether or not the amount of the protein secreted by the neuron in the presence of 7.5 mM glucose is at least 1.5 fold greater than the amount of the protein secreted by the neuron in the presence of 2.5 mM glucose.

### Determination of the cell number

Cell counting of control (untreated) and treated hmNPCs was performed using a hemocytometer. Adherent cells were collected by detachment with accutase^{™} (PAA Laboratories, Pasching, Austria) for 10 min at 37°C. To measure trypan blue exclusion (cell viability), cells were incubated in triplicate in 0.25% dye solution (Sigma). The number of total and trypan blue-positive cells was then determined by counting at least 300 cells per sample in a hemocytometer.

### Cell death /cell cycle assay

Untreated (control) and treated exponentially growing hmNPCs were prepared for cell cycle analysis by lysing cells in 300 µl of hypotonic lysis buffer (0.1% sodium citrate, 0.1% Triton X-100, and 50 µg/ml propidium iodide [PI]). The cells were subsequently analyzed by flow cytometry, on a FACScan (Becton Dickinson, Heidelberg, Germany), using 488 nm excitation, gating out doublets and clumps using pulse processing and collecting fluorescence above 620 nm according to the method of Nicoletti et al. (Nicoletti et al. 1991). Histograms of DNA content were acquired using the CellQuest software. The number of nuclei present in each peak of the histogram (sub-G1, G1, S, G2/M) was analyzed by measuring the peak area. For data analysis and correction of the background, noise histograms were processed with the ModFit LT software (Verity, Turramurra, Australia).

### Statistical analysis

Statistical analyses (one-way analysis of variance, ANOVA, followed by Tukey posthoc test) were performed using the SigmaStat software package (Jandel Corp., San Rafael, CA). Results are expressed as means ± SEM. Statistical significance was defined as P < 0.05.

### Transduction of glucose sensitive neurons

### Nucleofection protocol

- one nucleofection >4-5x106 cells (minimal cell number: 1x106 cells; maximum cell number: 6x106)
- sample contains >2-20 µg plasmid DNA (in 1-5 µl H2O or TE)
- resuspend the prepared accutase-treated cells in room temperature Mouse NSC Nucleofector
   (solution + DNA to a final concentration of 4-5x106 cells/100 µl)
- transfer the nucleofection sample into an amaxa certified cuvette and close cuvette with the blue cap.
- insert the cuvette into the cuvette holder and rotate the turning wheel clockwise to the final position
- select program A-33 or O-05
- take the cuvette out of the holder, transfer the cells from the cuvettes using the plastic pipettes provided in the kit to prevent damage and loss of cells and add 10 ml of the pre-warmed culture medium
- centrifuge the cells at 1500 rpm for 5 min at room temperature, add new culture medium
- transfer the sample into coated culture dishes, plates or flasks and incubate cells in a humidified 37°C/3% O2/5% CO2 incubator.
- replace culture medium after 24 h with either culture medium

control 1: Recommended amount of cells in Nucleofector Solution with DNA but without application of the program (alternatively: untreated cells) (Cells + Solution + DNA - program)
control 2: Recommended amount of cells in Nucleofector Solution without DNA with application of the program (Cells + Solution - DNA + program)

The vector used comprised a chicken-beta-actin-Promotor (see supra), an IRES-Site of the encephalomyocarditis virus (ECMV) between the MCS and the enhanced green fluorescent protein (EGFP) coding region, a sequence encoding EGFP, a Kan^{r}/Neo^{r} : neomycin-resistance cassette (Neo^{r}), a SV40 enhancer, an albumin leader sequence at the SIA to facilitate the secretion of SIA

### 2. Results

### 2.1. Generation of glucose sensitive neurons.

Glucose sensitive neurons were generated from NPCs grown with EGF and bFGF under low oxygen and low calcium conditions. NPCs were differentiated into glucose sensitive neurons via removal of mitogens and addition of nicotinamide (10 mM), B27-supplement (2%), N2-supplement (1%) and retinoic acid (2µM) or application of glucose waves. Reverse transciptase analysis of mRNA (figure 1) of 2 weeks differentiated insulin-producing cells resulted in a marked increase of the expression of the neuronal marker MAP-2 in N2-Nicotinamid-differentiated cells compared to Forskolin-induced differentiation of neuronal precurors. An increased expression was detected for the glucose-sensing markers GLUT2 and glucokinase with differentiation into insulin-producing cells using glucose waves or N2-Nicotinamid-differentiation compared to Forskolin-differentiated neuronal precursors. Insulin expression was detectable in N2-Nicotinamid-differentiated cells. Immunocytochemical staining (figure 3 and 4) showed high percentage of insulin-positive cells in N2-Nicotinamid-differentiated cultures. Additionally, 98,9 % of N2-Nicotinamid-differentiated neurons were insulin-positive. Insulin secretion could be increased by incubation with high glucose levels (7,5 mM and 25 mM) but not with 2 mM glucose.

### 2.2. Expression of heterologous protein

We first started to transfect glucose sensitive neurons with the marker protein "enhanced green fluorescent protein" (EGFP) using the nucleofection technology of AMAXA. Transduction efficiencies varied between 25 - 70 %. There was limited cell death (< 50 %) during the procedure. Resulting cells nicely expressed EGFP, which was released to the culture medium when increasing glucose concentrations were applied (figure 2).

### References

- Blyszczuk and Wobus, Methods Mol Biol 2006, 330: 373-385
- Buc-Caron, Neuroepithelial progenitor cells explanted from human fetal brain proliferate and differentiate in vitro, Neurobiol Dis 1995, 2:37-47
- Chalmers-Redman et al., In vitro propagation and inducible differentiation of multipotential progenitor cells from human fetal brain, Neuroscience 1997, 76:1121-1128
- Daadi und Weiss, Generation of tyrosine hydroxylase producing neurons from precurors of the embryonic and adult forebrain, J Neurosci 1999, 19:4484-4497
- Hermann et al., J Cell Sci. 2004, 117(Pt19):4411-4422
- Liepelt et al., Differentiation potential of a monoclonal antibody-defined neural progenitor cell population isolated from prenatal rat brain by fluorescence-activated cell sorting, Brain Res Dev Brain Res 1999, 51:267-278
- Ostenfeld et al., Exp Neurol. 2000, 164(1):215-226
- Physician's Guide to Insulin Dependent [Type I] Diabetes Mellitus: Diagnosis and Treatment, American Diabetes Association, 1988
- Ryan et al., Diabetes 2001, 50:710-719
- Sah et al. Biopotent progenitor cell lines from the human CNS ,Nat Biotechnol 1997, 15:574-580
- Scharp et al., Transplant 1991, 51:76
- Schwarz et al., Parkinsonism Relat Disord. 2006, 12(5):302-308,
- Shapiro et al., NEJN 2000; 343:230-238
- Svendsen et al. (Long-Term Survival of Human Central Nervous System Progenitor Cells Transplanted into a Rat Model of Parkinson's Disease, Exp Neurol 1997, 148:135-146
- UK prospective study group, 1995
- Vogel et al., Heterogeneity among human bone marrow-derived mesenchymal stem cells and neural progenitor cells, J. Haematol. 2003, 88, 126-133
- Warnock et al., Diabetologia 1991, 34:55
- Wikipedia, the free encyclopedia, 2006, http://en.wikipedia.org/wiki/Diabetes
- Wild et al., Diabetes Care 2004, 27: 1047-1053
- WO 2005/017132 A1 filed by NeuroProgen GmbH Leipzig
- WO 00/78931 A2, filed by NeuroProgen GmbH
- WO03/010304 A2 by NeuroProgen Leipzig GmbH

## Claims

1. A neuron comprising a cDNA encoding a protein, wherein the neuron is capable of secreting said protein in a glucose-dependent manner.

2. A neuron according to claim 1, **characterized in that** the amount of said protein secreted by the neuron in the presence of 7.5 mM glucose is at least 1.5fold greater than the amount of said protein secreted by the neuron in the presence of 2.5 mM glucose.

3. A neuron according to claim 1 or 2, wherein the neuron expresses the neuronal marker MAP-2 and at least one glucose-sensing marker selected from the group consisting of GLUT2, glucokinase and GLP-1 receptor..

4. A neuron according to claim 1 or 2, wherein the neuron expresses the neuronal marker Tuj1 and at least one glucose-sensing marker selected from the group consisting of GLUT2, glucokinase and GLP-1 receptor.

5. A neuron according to any one of claims 1 to 4, wherein said protein is insulin.

6. A neuron according to any one of claims 1 to 5, wherein said neuron does not express on its surface MHC-I molecules.

7. A neuron according to any one of claims 1 to 6, wherein said cDNA lacks a DNA sequence encoding the C-peptide of insulin.

8. A neuron according to any one of claims 1 to 7, wherein said cDNA encodes a single-chain insulin analogue (SIA).

9. A population of cells, **characterized in that** at least 10% of the cells in the population are neurons according to any one of claims 1 to 8.

10. A population of cells according to claim 9, **characterized in that** at least 20% of the cells in the population are neurons according to any one of claims 1 to 8.

11. A method for generating neurons that are capable of expressing a protein in a glucose-dependent manner, comprising the steps of
(a) culturing neural precursor cells in the presence of at least one factor capable of promoting islet cell development; and
(b) transfecting the cells with cDNA encoding said protein.

12. A method according to claim 11, **characterized in that** the cDNA encodes insulin.

13. A method according to claim 12, wherein the cDNA encodes an insulin analog lacking a C-peptide.

14. A method according to claim 12 or 13, wherein the cDNA encodes a single-chain insulin analogue (SIA).

15. A method according to any of claims 11 to 14, **characterized in that** the generated cells are glucose sensitive.

16. A method according to any of claims 11 to 15, **characterized in that** the factor capable of promoting islet cell development is selected from the group consisting of bFGF, EGF, Prolactin, Betacellulin, Glucagon, GLP-1 (Glucagon-like protein 1), Exendin-4, Nicotinamid, N2-supplement, retinoic acid, glucose waves, withdrawal of mitogens, and arginin.

17. A method according to claim 16, wherein step (a) comprises culturing the neural precursor cells in the presence of bFGF, N2-supplement, B27 supplement, nicotinamide and retinoic acid.

18. A method according to claim 17, wherein step (a) further comprises culturing the neural precursor cells in the presence of prolactin, betacellulin and/or GLP-1.

19. A method according to claim 17 or 18, wherein step (a) further comprises culturing the neural precursor cells in the presence of glucagons, exendin-4, IGF-1 and/or arginin.

20. A method according to any of claims 11 to 19, **characterized in that** the generated neurons express the neuronal marker(s) MAP2 and/or Tuj1.

21. A method according to any of claims 11 to 20, **characterized in that** the precursor cells are human precursor cells.

22. A method according to any of claims 11 to 21, **characterized in that** the method is carried out in vitro.

23. A population of cells obtainable by a method as defined in any one of claims 11 to 22.

24. The use of a neuron according to any one of claims 1 to 8 or of a population of cells according to any one of claims 9, 10 and 23 for the manufacture of a pharmaceutical composition for the treatment of a disorder.

25. The use according to claim 24, wherein the disorder is diabetes.

26. The use according to claim 13, wherein the diabetes is diabetes type I.


SEQUENCE LISTING

<110>  Innovalor AG

<120>  Method of generating glucose-responsive cells

<130>  glucose-responsive neurons

<160>  18

<170>  PatentIn version 3.3

<210>  1
<211>  24
<212>  DNA
<213>  artificial

<220>
<223>  Forward primer insulin

<400>  1
tcagcacctg tgcggctctc acct                                              24


<210>  2
<211>  23
<212>  DNA
<213>  artificial

<220>
<223>  Reverse primer insulin

<400>  2
cgatgccacg tttacccgga ccc                                               23


<210>  3
<211>  25
<212>  DNA
<213>  artificial

<220>
<223>  Forward primer MAP2

<400>  3
atcaaggcgg agcaggggga aggac                                             25


<210>  4
<211>  24
<212>  DNA
<213>  artificial

<220>
<223>  Reverse primer MAP2

<400>  4
tgggggtgga gaaggaggca gatt                                              24


<210>  5
<211>  21
<212>  DNA
<213>  artificial

<220>
<223>  Forward primer Glut2

<400>  5

ggtttgtaac ttatgcctaa g                                                21

<210>  6
<211>  19
<212>  DNA
<213>  artificial

<220>
<223>  Reverse primer Glut2

<400>  6
gcctagttat gcattgcag                                                   19

<210>  7
<211>  23
<212>  DNA
<213>  artificial

<220>
<223>  Forward primer Glucokinase

<400>  7
gaataccccc cagagacctt ttc                                              23

<210>  8
<211>  20
<212>  DNA
<213>  artificial

<220>
<223>  Reverse primer Glucokinase

<400>  8
ggtttcttcc tgagccagcg                                                  20

<210>  9
<211>  20
<212>  DNA
<213>  artificial

<220>
<223>  Forward primer GAPDH

<400>  9
gtcagtggtg gacctgacct                                                  20

<210>  10
<211>  20
<212>  DNA
<213>  artificial

<220>
<223>  Reverse primer GAPDH

<400>  10
tgctgtagcc aaattcgttg                                                  20

<210>  11
<211>  20
<212>  DNA
<213>  artificial

<220>

<223>  Forward primer GLP-1 receptor

<400>  11
ttctgcaacc ggaccttcga                                                    20


<210>  12
<211>  21
<212>  DNA
<213>  artificial

<220>
<223>  Reverse primer GLP-1 receptor

<400>  12
atgagtgtca gcgtggactt g                                                  21


<210>  13
<211>  20
<212>  DNA
<213>  artificial

<220>
<223>  Forward primer Nestin

<400>  13
cgttggaaca gaggttggag                                                    20


<210>  14
<211>  20
<212>  DNA
<213>  artificial

<220>
<223>  Reverse primer Nestin

<400>  14
taagaaaggc tggcacaggt                                                    20


<210>  15
<211>  20
<212>  DNA
<213>  artificial

<220>
<223>  Forward primer Beta-III-tubulin

<400>  15
caacagcacg gccatccagg                                                    20


<210>  16
<211>  21
<212>  DNA
<213>  artificial

<220>
<223>  Reverse primer Beta-III-tubulin

<400>  16
cttggggccc tgggcctccg a                                                  21


<210>  17
<211>  183
<212>  DNA

<213>  artificial

<220>
<223>  DNA encoding single-chain insulin analogue


<220>
<221>  CDS
<222>  (1)..(183)

<400>  17
atg ttc gtt aat cag cac ctg tgc ggc tct cac ctg gta gaa gct ctg      48
Met Phe Val Asn Gln His Leu Cys Gly Ser His Leu Val Glu Ala Leu
1               5                   10                  15

tac ctg gtt tgc ggt gaa cgt ggt ttt ttc tac acc ccg aaa acc ggt      96
Tyr Leu Val Cys Gly Glu Arg Gly Phe Phe Tyr Thr Pro Lys Thr Gly
            20                  25                  30

ggt ggt ccg ggt aaa cgt ggc atc gtt gaa caa tgc tgt act agc atc      144
Gly Gly Pro Gly Lys Arg Gly Ile Val Glu Gln Cys Cys Thr Ser Ile
        35                  40                  45

tgc tct ctc tac cag ctg gag aac tat tgt aac tag taa                  183
Cys Ser Leu Tyr Gln Leu Glu Asn Tyr Cys Asn
    50                  55


<210>  18
<211>  59
<212>  PRT
<213>  artificial

<220>
<223>  Synthetic Construct

<400>  18

Met Phe Val Asn Gln His Leu Cys Gly Ser His Leu Val Glu Ala Leu
1               5                   10                  15


Tyr Leu Val Cys Gly Glu Arg Gly Phe Phe Tyr Thr Pro Lys Thr Gly
            20                  25                  30


Gly Gly Pro Gly Lys Arg Gly Ile Val Glu Gln Cys Cys Thr Ser Ile
        35                  40                  45


Cys Ser Leu Tyr Gln Leu Glu Asn Tyr Cys Asn
    50                  55